# EUROPEAN PATENT SPECIFICATION

(11) **EP 0 500 835 B1**
(45) Date of publication and mention of the grant of the patent: **27.12.1996**
(21) Application number: 91914696.9
(22) Date of filing: 14.08.1991
(51) Int. Cl.: C07D 401/04, C07D 203/06, C07D 207/08, C07D 207/12, C07D 211/20, C07D 211/54, A61K 31/445

(54) **A NOVEL QUINOLONE CARBOXYLIC ACID DERIVATIVE**
EIN NEUES CHINOLON-CARBONSÄUREDERIVAT
NOUVEAUX DERIVES D'ACIDE CARBOXYLIQUE DE QUINOLONE

(30) Priority: 04.09.1990 KR 9013937; 25.06.1991 KR 9110561
(43) Date of publication of application: 02.09.1992
(73) Proprietor: DAEWOONG PHARMACEUTICAL CO., LTD., Sungnam, Kyonggi-do 461-121 (KR)
(72) Inventor: YOON, Geal, Jung, 102-103 Sungji APT., Kyonggi-Do 461-121 (KR); KIM, Dae, Young, Na-202 Taeyangyunlip, Kyonggi-Do 461-121 (KR); LEE, Jae, Wook, 107-410 Sungji APT., Kyonggi-do 461-121 (KR); PARK, Nam, Jun, 103-1402 Jukong APT., Kyonggi-Do 461-121 (KR); LEE, Kyeu, Sam, 51-2, Singongdug-Dong, Seoul 121-030 (KR); KANG, Tae, Choong, 108-402 Sungji APT., Kyonggi-Do 461-121 (KR)
(74) Representative: Weber, Dieter, Dr.
(86) International application number: KR9100018
(87) International publication number: WO9204342

(56) References cited:
- EP-A- 0 366 643
- EP-A- 0 401 623
- DE-A- 3 517 535
- DE-A- 3 906 365
- CHEMICAL ABSTRACTS, Volume 104, No.11, issued 17 March 1986, (Columbus, Ohio, US), H. BARRERA et al., "Synthesis and spectroscopic characterization of some gamma-mercaptoalkylpiperidines", see page 644, column 1, the Abstract No. 88398k, J. Chem. Res., Synop. 1985, (8), 270-1.
- CHEMICAL ABSTRACTS, Volume 104, no. 11, issued 17 March 1986, (Columbus, Ohio, US), M. HASHIMOTO et al., "3-Mercaptopyrrolidines and their salts", see page 646, column 2, the Abstract No. 88425s, Jpn. Kokai Tokyo Koho JP 60,178,860 (85,178,860).

## Description

The invention relates to novel quinolone carboxylic acid derivatives and pharmaceutically acceptable salts thereof.

The EP-A-0 401 623, the DE-A-3 906 365 and the DE-A-3 517 535 disclose quinoline carboxylic acid derivatives having the same ring system as the compounds of the invention with 3-pyrrolidinyl substitution in 7-position, but with other substitution of the 3-pyrrolidinyl ring.

The WO-A-89/05 806 and Chemical Abstracts, 104 (1986), Abstract 88425s and Abstract 88398k, describe the production of 3-mercapto pyrrolidines and their salts which compounds may be used as starting materials for the preparation of the compounds according to the invention.

In general, the antibacterial activity of quinolone compounds depends greatly upon the kind of substituent groups at the 7-position. Quinolone antibacterial compounds are known with a 7-thiomorpholine substituent group having antibacterial properties (J. Med. Chem. V.29, 394 [1986]). But there is no report on clinical use thereof yet.

Therefore, the object of the present invention is to provide novel quinolone carboxylic acid derivatives and pharmaceutically acceptable salts thereof having excellent properties, for example, potent antibacterial activities against Gram-positive bacteria including S. aureus as well as against Gram-negative bacteria and high safety. This object is solved by the compounds according to claim 1 and the pharmaceutical compositions according to claim 9. Preferred embodiments are shown by the subclaims. These compounds show a very excellent activity against Gram-positive bacteria including S. aureus. as well as against Gram-negative bacteria and a low toxicity.

The preferred examples of the group represented by Z in the above described structural formula(I) are as follows :
3-mercaptomethylpyrrolidinyl,
3-methylthiomethylpyrrolidinyl,
3-ethylthiomethylpyrroIidinyl,
3-propylthiomethylpyrrolidinyl,
3-isopropylthiomethylpyrrolidinyl,
3-phenylthiomethylpyrrolidinyl,
3-(2-pyfidylthiomethyl)-pyrrolidinyl,

Also preferred group of compounds of this invention is compounds of the above formula(I) wherein R² is ethyl, fluoroethyl, cyclopropyl, tert-butyl, 2,4-difluorophenyl, and 4-fluorophenyl radical.

Other preferred compounds of this invention are those wherein X is hydrogen or amino, Y is hydrogen, fluorine, or chlorine, and R¹ is hydrogen. Representative examples of the novel compound of the structural formula(I) according to the present invention can be given as follows, but the present invention is not limited to the examples given as follows :
1-cyclopropyl-6-fluoro-7-(3-methylthiomethylpyrrolidinyl)-1,4-dihydro-4-oxo-3-quinolinecarboxylic acid,
1-cyclopropyl-6,8-difluoro-7-(3-methylthiomethylpyrrolidinyl)-1,4-dihydro-4-oxo-3-quinolinecarboxylic acid,
1-cyclopropyl-5,6,8-trifluoro-7-(3-methylthiomethylpyrrolidinyl)-1,4-dihydro-4-oxo-3-quinolinecarboxylic acid,
1-cyclopropyl-6,8-difluoro-7-(3-ethylthiomethylpyrrolidinyl)-1,4-dihydro-4-oxo-3-quinolinecarboxylic acid,
1-cyclopropyl-5,6,8-trifluoro-7-(3-ethylthiomethylpyrrolidinyl)-1,4-dihydro-4-oxo-3-quinolinecarboxylic acid,
1-cyclopropyl-6-fluoro-8-chloro-7-(3-methylthiomethylpyrrolidinyl)-1,4-dihydro-4-oxo-3-quinolinecarboxylic acid,
1-(2,4-difluorophenyl)-6,8-difluoro-7-(3-methylthiomethylpyrrolidinyl)-1,4-dihydro-4-oxo-3-quinolinecarboxylic acid,
1-(2,4-difluorophenyl)-5,6,8-trifluoro-7-(3-ethylthiomethylpyrrolidinyl)-1,4-dihydro-4-oxo-3-quinolinecarboxylic acid,
1-cyclopropyl-5-amino-6,8-difluoro-7-(3-methylthiomethylpyrrolidinyl)-1,4-dihydro-4-oxo-3-quinolinecarboxylic acid,
1-cyclopropyl-6,8-difluoro-7-(3-mercaptomethylpyrrolidinyl)-1,4-dihydro-4-oxo-3-quinolinecarboxylic acid,
1-cyclopropyl-6,8-difluoro-7-[3-(2-pyridylthiomethyl)-pyrrolidinyl]-1,4-dihydro-4-oxo-3-quinolinecarboxylic acid,
1-(2,4-difluorophenyl)-6,8-difluoro-7-[3-(2-pyridylthiomethyl)-pyrrolidinyl]-1,4-dihydro-4-oxo-3-quinolinecarboxylic acid,
1-cyclopropyl-6,8-difluoro-7-(3-phenylthiomethylpyrrolidinyl)-1,4-dihydro-4-oxo-3-quinolinecarboxylic acid,
1-(2,4-difluorophenyl)-6-fluoro-7-(3-phenylthiomethylpyrrolidinyl)-1,4-dihydro-4-oxo-3-quinolinecarboxylic acid.

The compounds of the formula(I) of the present invention can be prepared by reacting a quinolone carboxylic acid of the following structural formula(II) with an amine of the following structural formula(III) in an inert solvent or a basic solvent. 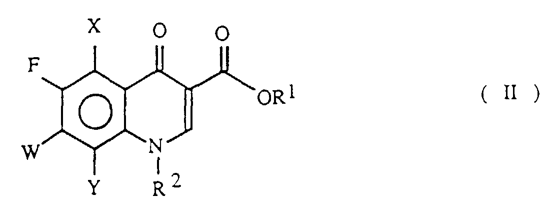 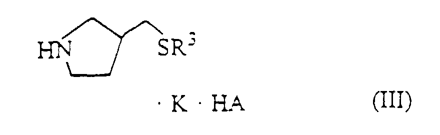 wherein:
R¹, R², R³, X, Y are as defined above,
W is halogen (preferably, fluorine or chlorine),
HA is an inorganic or organic acid capable of forming a salt with amines,
K is 0 or 1.

Convenient solvents for this reaction are non-reactive solvents such as tetrahydrofuran, pyridine, ethanol, propanol, butanol, chloroform, dimethylsulfoxide, dimethylformamide, water, acetonitrile, dioxane .

Solvent mixtures may also be utilized. The reaction is preferably carried out in the presence of an acid acceptor such as an alkali metal or alkaline earth metal carbonate, alkali metal or alkaline earth metal bicarbonate, or a tertiary amine such as triethylamine, 1,8-diazabicyclo [5.4.0]undec-7-ene(DBU), pyridine.

According to the present invention, the preferred solvent is acetonitrile and the preferred base is 1,8-diazabicyclo[5.4.0]undec-7-ene(DBU), triethylamine, and mixture thereof.

The reaction temperature ranges from 50 °C to 190°C, preferably from 50 °C to 120°C at atmospheric pressure. The reaction is usually carried out for about 1 hour to about 48 hours.

According to another method, the compound of the formula(I) can be prepared by reacting a boron complex, the formula(IV), with the compound of the above-described formula(III) and then hydrolyzing the reaction product. 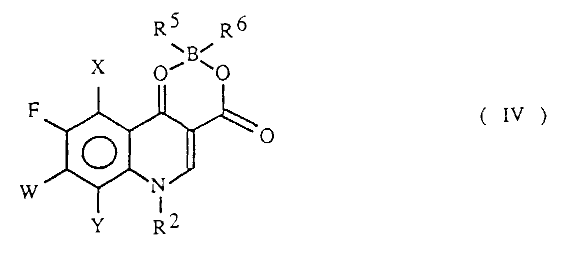 wherein,
- R², X, Y, and W: are as defined above,
- R⁵ and R⁶: is aliphatic acyloxy group having two to six carbon atoms or aromatic acyloxy group having seven to eleven carbon atoms.

The starting compounds having structural formula(II) are known in the article (European Patent Application No. 78362, No. 106489, No. 113091, No. 131839, No. 153580, No. 154780, No. 202763, No. 221463, No. 235762, No. 350950, and No. 360258),

The amine compound of the above-described structural formula(III) can be prepared through the following reaction sequence from the known starting material 3-hydroxymethyl pyrrolidine (J. Org. Chem., V. 26, 1519(1961)). 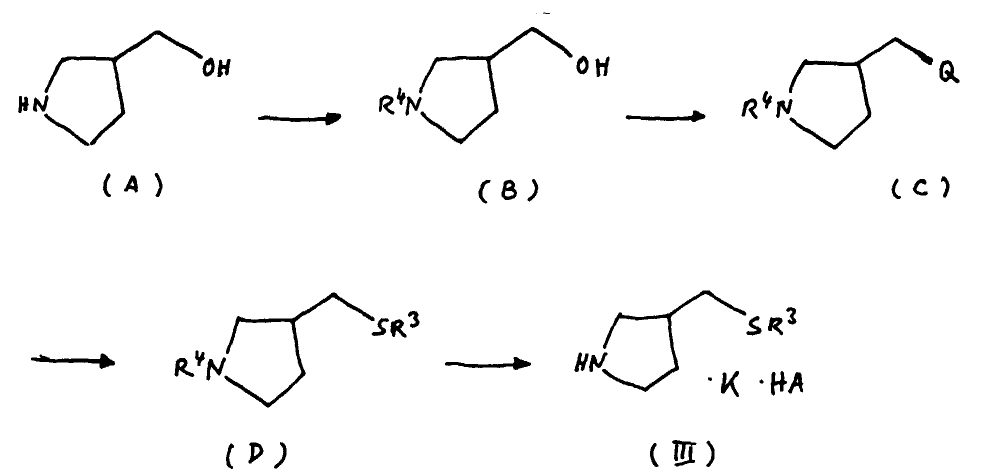 wherein,
- R³: is alkyl group having one to four carbon atoms, haloalkyl group having one to three carbon atoms, cycloalkyl group having three to six carbon atoms, phenyl or 2-pyridinyl group.
- R⁴: is amine phenyl protecting group,
- Q: is alkylsulfonyloxy group, arylsulfonyloxy group, or halide,
- HA: is organic acid or inorganic acid.
- K: is 0 or 1,

Thus compound(A) may be converted to compound(B) by treatment with an appropriate amine protection reagent. For example, a known amine protection reagent, preferably, acetic anhydride, alkoxycarbonyl anhydride, acetyl halide, or alkoxycarbonyl halide may be utilized.

The hydroxyl function of the compound(B) may next be converted to an appropriate leaving group of compound(C) by treatment with convinient reagents, for example, methanesulfonyl chloride or p-toluenesulfonyl chloride in the presence of a base(for example, triethylamine, pyridine), or thionylchloride or phosphorous trihalide.

Compound(C) may also be converted to compound(D) by treatment with thioalkoxide or mercapto compound in the presence of an appropriate base (for example, sodium hydride, potassium ten-butoxide).

Finally, compound(D) may be deprotected to produce compound of the above described structural formula(III) in the state of an acid salt or a free base by various well known reagents, for example, hydrochloric acid, trifluoroacetic acid, methanesulfonic acid, iodotrimethylsilane, sodium hydroxide.

On the other hand, pharmaceutically acceptable acid addition salts of the above-described structural formula(I) are formed with organic acid or inorganic acids.

Examples of suitable acids for salt formation are acetic acid, lactic acid, succinic acid, maleic acid, tartaric acid, citric acid, gluconic acid, ascorbic acid, benzoic acid, methanesulfonic acid, cinnamic acid, fumaric acid, phosphoric acid, hydrochloric acid, hydroiodic acid, sulfuric acid.

The salts are prepared by contacting the free base form with a sufficient amount of the desired acid to produce either a mono or di, salt in the conventional manner.

Pharmaceutically acceptable base salts of the above-described structural formula(I) are formed with metals such as alkali and alkaline earth metals , or amines including ammonia and organic amines.

Examples of metals used as cations are sodium, potassium, magnesium, calcium. Examples of suitable amines are diethanolamine, N-methylglucamine, arginine.

The compounds of the present invention can be prepared and administered in a wide variety of oral and parenteral dosage forms.

For preparing pharmaceutical compositions from the compounds described by this invention, inert and pharmaceutically acceptable corners can be either solid or liquid. Solid form preparations include powders, tablets, dispersable granules, capsules, cachets, suppositories, and ointments. A solid carriers can be one or more substances which may also act as diluents, flavoring agents, solubilizers, lubricants, suspending agents, binders, or tablets disintegrating agents ; it can also be an encapsulating material.

Suitable solid carriers are magnesium carbonate, magnesium sterate, talc, sugar, lactose, pectin, dextrin, starch, gelatin, tragacanth, methyl cellulose, sodium carboxymethyl cellulose.

Liquid form preparations include solutions, suspensions and emulsions. As an example may be mentioned water or water-propylene glycol solutions for parenteral injection. Such solutions are prepared so as to be acceptable to biological systems(isotonicity, pH,). Liquid preparations can also be formulated in solution in aqueous polyethylene glycol solution.

The following examples illustrate the inventors' preferred methods for preparing the compounds of the invention.

### Reference Example 1

### Preparation of N-tert-butoxycarbonyl-3-hydroxymethylpyrrolidine

A mixture of 3.5g (0.035 mole) of 3-hydroxymethylpyrrolidine[J. Org. Chem., 26, 1519(1961)] in 90*ml* of dioxane, 40*ml* of 1N NaOH, and 20*ml* of water was cooled to 0°C, treated dropwise with a solution of 9g (0.041 mole) of di-tert-butyldicarbonate in 30*ml* of dioxane, and warmed to room temperature. The resulting solution was stirred for 12 hours and extracted with 40*ml* of ethyl acetate over three times. The organic layer was dried over anhydrous magnesium sulfate, filtered and evaporated under reduced pressure to afford 5.0g of the title compound as yellowish brown oil.
- ¹H NMR(ppm, CDCl₃) :: 1.45(s, 9H), 1.56 ∼ 2.47(m, 3H), 2.97 ∼ 3.61(m, 6H).

### Reference Example 2

### Preparation of N-tert-butoxycarbonyl-3-hydroxypyrrolidine

In the same manner as described in Reference Example 1, except that 3-hydroxypyrrolidine was used in place of 3-hydroxymethylpyrrolidine, the tide compound was prepared as yellowish brown oil.
- ¹H NMR(ppm, CDCl₃) :: 1.44(s, 9H), 1.85 ∼ 1.94(m, 2H), 3.35 ∼ 3.54(m, 4H), 3.74(s, 1H), 4.40(m, 1H).

### Reference Example 3

### Preparation of N-tert-butoxycarbonyl-3-[(methylsulfonyl)-oxymethyl]-pyrrolidine

A solution of 5g(0.025 mole) of N-tert-butoxycarbonyl-3-hydroxymethylpyrrolidine in 80*ml* of dichloromethane was treated with 4*ml* (0.029mole) of triethylamine and cooled to -5 °C to -10°C.

A solution of 3.3g(0.029 mole) of methanesulfonyl chloride in 20*ml* of dichloromethane was added dropwise to the reaction mixture, stirred for 10 hours at room temperature, and then treated with 80*ml* of water. After 30 minutes, the organic layer was separated, washed with 10% NaHCO₃ solution, dried over anhydrous magnesium sulfate, filtered, and evaporated under reduced pressure to give the crude product. This was purified by column chromatography using ethyl acetate : dichloromethane (1 : 1) as an eluent to afford 5.9g of the title compound as yellowish brown oil.
- ¹H NMR(ppm, CDCl₃) :: 1.45(s, 9H), 1.48 ∼ 2.70(m, 3H), 3.03(s, 3H), 3.10 ∼ 3.57(m, 4H), 4.20(dd, 2H).

### Reference Example 4

### Preparation of 3-methylthiomethylpyrrolidine hydroiodide

A solution of 2.8g(0.01 mole) of N-tert-butoxycarbonyl-3-[(methylsulfonyl) -oxymethyl] pyrrolidine and 0.74g(0.01 mole) of sodium thiomethoxide in 30*ml* of anhydrous dimethylformamide was stirred for 4 hours at 80°C and cooled to room temperature. The reaction mixture was poured into 50*ml* of water and extracted with 50*ml* of diethyl ether over twice. The organic layer was dried over magnesium sulfate and evaporated under reduced pressure to give the crude product. This was purified by column chromatography using diethyl ether : n-hexane (4 : 1) as an eluent to afford 2.05g of N-tert-butoxycarbonyl-3-methylthiomethylpyrrolidine as yellowish oil.
- ¹H NMR(ppm, CDCl₃) :: 1.45(s. 9H), 1.61 ∼ 2.36(m, 3H), 2.12(s, 3H), 2,51(d, 2H), 2.81 ∼ 3.82(m, 4H).

A solution of 2.05g (8.64 mmole) of N-tert-butoxycarbonyl-3-methylthiomethylpyrrolidine in 20*ml* of chloroform was added dropwise with 1.6*ml* (11.2 mmole) of iodotrimethylsilane at 5°C under N₂.

The resulting solution was stirred at room temperature. After 2 hours, 10 *ml* of methanol was added to the reaction mixture. Then the removal of solvent under reduced pressure was afforded 2.34g of the title compound as brown oil.
- ¹H NMR(ppm, CDCl₃) :: 1.52 ∼ 2.46(m, 3H), 2.13(s, 3H), 2.59(d, 2H), 2.62 ∼ 3.41(m, 4H), 7.02(br, 2H).

### Reference Example 5

### Preparation of 3-ethylthiomethylpyrrolidine hydroiodide

In the same manner as described in Reference Example 9, except that sodium thioethoxide was used in place of sodium thiomethoxide, the title compound was prepared as yellowish brown oil.
- ¹H NMR(ppm, CDCl₃) :: 1.28(t, 3H), 1.51 ∼ 2.60(m, 5H), 2.59(d, 2H), 2.63 ∼ 3.51(m, 4H), 8.86(br, 2H).

### Reference Example 6

### Preparation of N-tert-butoxycarbonyl-3-mercaptomethylpyrrolidine

To a solution of 0.3g(1.16 mmole) of N-tertbutoxycarbonyl-3-methylcarbonylthiomethylpyrrolidine in 10*ml* of ethanol was added 10*ml* of 1N NaOH. The resulting mixture was stirred for 6 hours at 60°C, cooled to room temperature, evaporated under reduced pressure, and treated 20*ml* of water. Then the resulting solution was extracted with 20*ml* of diethyl ether over twice. The organic layer was dried over magnesium sulfate, filtered, and evaporated under reduced pressure to afford 0.23g of the title compound as yellowish brown oil.
- ¹H NMR(ppm, CDCl₃) :: 1.46(s, 9H), 1.73 ∼ 2.72(m, 3H), 2.70(s, 2H), 2.84 ∼ 3.90(m, 4H).

### Reference Example 7

### Preparation of 3-mercaptomethylpyrrolidine trifluoroacetate

To a mixture of 0.1g of anisole and 0.25g of N-tert-butoxycarbonyl-3-mercaptomethylpyrrolidine was added 1*ml* of trifluoroacetic acid at 0°C. The mixture was stirred for 2 hours at room temperature. The reaction mixture was evaporated under reduced pressure followed by washing with 20*ml* of n-hexane over twice. This was concentrated under reduced pressure to afford 0.25g of the title compound as yellowish brown oil.
- ¹H NMR(ppm, CDCl₃) :: 1.54 - 2.42(m, 3H), 2,72(d, 2H), 2.92 ∼ 3.64(m, 5H), 8.24(br, 2H).

### Reference Example 8

### Preparation of N-tert-butoxycarbonyl-3-(2-pyridylthiomethyl)-pyrrolidine

A solution of 0.42g(3.77 mmole) of 2-mercaptopyridine in 10*ml* of anhydrous dimethyl formamide was added dropwise to a solution of 0.15g(3.75 mmole) of 60% sodium hydride in 20*ml* of anhydrous dimethylformamide at -5°C to 0°C and stirred for 1 hour at room temperature. To the resulting solution was added a solution of 1 g(3.58 mmole) of N-tert-butoxycarbonyl-3-[(methylsulfonyl)-oxymethyl]pyrrolidine in 10*ml* of dimethylformamide. The reaction mixture was stirred for 6 hours at 80°C to 100°C, cooled to room temperature, added 80*ml* of water, and extracted with 50*ml* of diethyl ether over twice. The organic layer was dried over magnesium sulfate and evaporated under reduced pressure to give the crude product. This was purified by column chromatography using isopropyl ether as an eluent to afford 0.94g of the title compound as yellowish brown oil.
- ¹H NMR(ppm, CDCl₃) :: 1,45(s, 9H), 1.64 ∼ 2.70(m, 3H), 2.95(d, 2H), 3.0 ∼ 3.70(m, 4H), 6.90 ∼ 7.18(m, 3H), 8.43(dd, 1H).

### Reference Example 9

### Preparation of N-tert-butoxycarbonyl-3-phenylthiomethylpyrrolidine

In the same manner as described in Reference Example 19, except that thiophenol was used in place of 2-mercaptopyridine, the title compound was prepared as yellowish brown oil.
- ¹H NMR(ppm, CDCl₃) :: 1.44(s,9H), 1.60 ∼ 2.62(m, 3H), 2.97(d, 2H), 3.10 ∼ 3,72(m, 4H), 7.30(s, 5H).

### Reference Example 10

### Preparation of 3-(2-pyridylthiomethyl)-pyrrolidine trifluoroacetate

In the same manner as described in Reference Example 18, except that N-tert-butoxycarbonyl-3-(2-pyridylthiomethyl)-pyrrolidine was used in place of N-tert-butoxycarbonyl-3-mercaptomethylpyrrolidine, the title compound was prepared as brown oil.
- ¹H NMR(ppm, CDCl₃) :: 1.51 ∼ 2.62(m, 3H), 3.04(d, 2H), 3.16 ∼ 4.01(m, 4H), 7.20 ∼ 9.20(m, 4H), 9.12(br,2H).

### Reference Example 11

### Preparation of 3-phenylthiomethylpyrrolidine trifluoroacetate

In the same manner as described in Reference Example 18, except that N-tert-butoxycarbonyl-3-phenylthiomethylpyrrolidine was used in place of N-tert-butoxycarbonyl-3-mercaptomethylpyrrolidine, the title compound was prepared as brown oil.
- ¹H NMR(ppm, CDCl₃) :: 1.54 ∼ 2.70(m, 3H), 2.95(d, 2H), 3.01 ∼ 3.70(m, 4H), 7.30(s, 5H), 8.77(br, 2H).

### Example 1

### Preparation of 1-cyclopropyl-6-fluoro-7-(3-methylthiomethylpyrrolidinyl)-1,4-dihydro-4-oxo-3-quinolinecarboxylic acid

A mixture of 0.2g(0.71 mmole) of 1-cyclopropyl-7-chloro-6-fluoro-1,4-dihydro-4-oxo-3-quinolinecarboxylic acid, 0.9g(3.5 mmole) of 3-methylthiomethylpyrrolidine hydroiodide, and 0.4g(3.96 mmole) of triethylamine in 6*ml* of dimethylsulfoxide was stirred for 6 hours at 110°C and cooled to room temperature.

The reaction mixture was diluted with 15*ml* of water, adjusted at pH 7.4 with 1N HCl, and then allowed to stand for 1 day. The precipitated product was filtered, washed with water and methanol, and recrystallized from methanol : dichloromethane(1:1) to afford 0.19g of the title compound as pale yellow solid.
- m.p. :: 196 ∼ 198°C
- ¹H NMR(ppm, CDCl₃) :: 1.19 ∼ 1.31(m, 4H), 1.50 ∼ 2.50(m, 3H), 2.17(s, 3H), 2.67(d, 2H), 3.20 ∼ 4.01(m, 5H), 6.84(d, 1H), 7.80(d, 1H), 8.59(s, 1H), 15.29(br, 1H).

### Example 2

### Preparation of 1-cyclopropyl-6,8-difluoro-7-(3-methylthiomethylpyrrolidinyl)-1,4-dihydro-4-oxo-3-quinolinecarboxylic acid

A mixture of 0.1g(0.35 mmole) of 1-cyclopropyl-6,7,8-trifluoro-1,4-dihydro-4-oxo-3-quinolinecarboxylic acid 0.06g(0.39 mmole) of 1,8-diazabicyclo [5.4.0]undec-7-ene, 0.04g(0.39 mmole) of triethylamine, and 0.1g(0.39 mmole) of 3-methylthiomethylpyrrolidine hydroiodide in 6*ml* of acetonitrile was stirred for 3 hours at 60°C, cooled to room temperature, and stirred for 2 hours. The precipitate was filtered, washed with acetonitrile and diethyl ether, and dried to afford 0.12g of the title compound as pale yellow solid.
- m.p. :: 166 ∼ 168°C
- ¹H NMR(ppm, CDCl₃) :: 1.10 ∼ 1.30(m, 4H), 1.54 ∼ 2.42(m, 3H), 2.16(s, 3H), 2.62(d, 2H), 3.34 ∼ 4.20(m, 5H), 7.80(dd, 1H), 8.68(s, 1H), 14.01(br, 1H)

### Example 3

### Preparation of 1-cyclpropyl-5,6,8-trifluoro-7-(3-methylthiomethylpyrrolidinyl)-1,4-dihydro-4-oxo-3-quinolinecarboxylic acid

A mixture of 0.2g(0.66 mmole) of 1-cyclopropyl-5,6,7,8-tetrafluoro-1,4-dihydro-4-oxo-3-quinolinecarboxylic acids 0.1g(0.66 mmole) of 1,8-diazabicyclo [5.4.0]undec-7-ene, 0.07g(0.67 mmole) of triethylamine, and 0.18g(0.69 mmole) of 3-methylthiomethylpyrrolidine hydroiodide in 6*ml* of acetonitrile was stirred for 3 hours at 60°C, cooled to room temperature, and stirred for 3 hours.

The precipitate was filtered, washed with acetonitrile and diethyl ether, and dried to afford 0.21g of the title compound as pale yellow solid.
- m.p. :: 200 ∼ 204°C
- ¹H NMR(ppm, CDCl₃) :: 1.17 ∼ 1.27(m, 4H), 1.76 ∼ 2.49(m, 3H), 2.17(s, 3H), 2,63(d, 2H), 3.54 ∼ 4.04(m, 5H), 8.63(s, 1H), 14.31(br, 1H)

### Example 4

### Preparation of 1-cyclopropyl-6,8-difluoro-7-(3-ethylthiomethylpyrrolidinyl)-1,4-dihydro-4-oxo-3-quinolinecarboxylic acid

In the same manner as described in Example 9, except that 3-ethylthiomethylpyrrolidine hydroiodide was used in place of 3-methylthiomethylpyrrolidine hydroiodide, the title compound was prepared as pale yellow solid.
- ¹H NMR(ppm, CDCl₃) :: 1.12 ∼ 1.43(m, 7H), 1.64 ∼ 2.38(m, 3H), 2.42 ∼ 2.78(m, 4H), 3.32 - 4,26(m, 5H), 7.82(dd, 1H), 8.67(s, 1H), 14.26(br, 1H)

### Example 5

### Preparation of 1-cyclopropyl-5,6,8-trifluoro-7-(3-ethylthiomethylpyrrolidinyl)-1,4-dihydro-4-oxo-3-quinolinecarboxylic acid

In the same manner as described in Example 10, except that 3-ethylthiomethylpyrrolidine hydroiodide was used in place of 3-methylthiomethylpyrrolidine hydroiodide, the title compound was prepared as pale yellow solid.
- ¹H NMR(ppm, CDCl₃) :: 1.17 ∼ 1.46(m, 7H), 1.58 ∼ 2.42(m, 3H), 2.41 ∼ 2.80(m, 4H), 3.26 ∼ 4.24(m, 5H), 8.63(s, 1H), 14.42(br, 1H)

### Example 6

### Preparation of 1-cyclopropyl-6-fluoro-8-chloro-7-(3-methylthiomethylpyrrolidinyl)-1,4-dihydro-4-oxo-3-quinolinecarboxylic acid

In the same manner as described in Example 9, except that 1-cyclopropyl-6,7-difluoro-8-chloro-1,4-dihydro-4-oxo-3-quinolinecarboxylic acid was used in place of 1-cyclopropyl-6,7,8-trifluoro-1,4-dihydro-4-oxo-3-quinolinecarboxylic acid, the title compound was prepared as pale yellow solid
- ¹H NMR(ppm, CDCl₃) :: 1.12 ∼ 1.32(m, 4H), 1.52 ∼ 2.43(m, 3H), 2.16(s, 3H), 2.61(d, 2H), 3.32 ∼ 4.16(m, 5H), 7.84(dd, 1H), 8.64(s, 1H), 14.38(br, 1H)

### Example 7

### Preparation of 1-(2,4-difluorophenyl)-6,8-difluoro-7-(3-methylthiomethylpyrrolidinyl)-1,4-dihydro-4-oxo-3-quinolinecarboxylic acid

A mixture of 0.2g(0.56 mmole) of 1-(2,3-difluorophenyl)-6,7,8-trifluoro-1,4-dihydro-4-oxo-3-quinolinecarboxylic acid, 0.09g(0.59 mmole) of 1,8-diazabicyclo[5.4.0]undec-7-ene. 0.06g(0.59 mmole) of triethylamine, and 0.15g(0.58 mmole) of 3-methylthiomethylpyrrolidine hydroiodide in 8*ml* of acetonitrile was stirred for 4 hours at 60°C, cooled to room temperature, stirred for 2 hours, and evaporated under reduced pressure.

The mixture was diluted with 20*ml* of water. The precipitate was filtered and recrystallized from methanol : dichloromethane(1:1) to afford 0.2g of the title compound as pale yellow solid.
- m.p. :: 178∼182°C
- ¹H NMR(ppm, CDCl₃) :: 1.47 ∼ 2.34(m, 3H), 2.14(s, 3H), 2.60(d, 2H), 3.60 ∼ 3.78(m, 4H), 6.95 ∼ 7.52(m, 3H), 7.75(dd, 1H), 8.41(s, 1H), 14.54(br, 1H)

### Example 8

### Preparation of 1-(2,4-difluorophenyl)-5,6,8-trifluoro-7-(3-methylthiomethylpyrrolidinyl)-1,4-dihydro-4-oxo-3-quinolinecarboxylic acid

A mixture of 0.2g(0.54 mmole) of 1-(2,4-difluorophenyl)-5,6,7,8-tetrafluoro-1,4-dihydro-4-oxo-3-quinolinecarboxylic acid, 0.09g(0.59 mmole) of 1,8-diazabicyclo[5.4.0]undec-7-ene, 0.06g(0.59 mmole) of triethylamine, and 0.15g(0.58 mmole) of 3-methylthiomethylpyrrolidine hydroiodide in 10*ml* of acetonitrile was stirred for 4 hours at 60°C, cooled to room temperature, stirred for 3 hours, and evaporated under reduced pressure.

The mixture was diluted with 20*ml* of water. The precipitate was filtered and recrystallized from methanol: dichloromethane(1 : 1) to afford 0.2g of the title compound as pale yellow solid.
- m.p. :: 190 ∼ 194°C
- ¹H NMR(ppm, CDCl₃) :: 1.48 ∼ 2.43(m, 3H), 2.12(s, 3H), 2.64(d, 2H), 3.43 ∼ 3.99(m, 4H), 6.94 ∼ 7.50(m, 3H), 8.36(s, 1H), 14.49(br, 1H)

### Example 9

### Preparation of 1-cyclopropyl-5-amino-6,8-difluoro-7-(3-methylthiomethylpyrrolidinyl)-1,4-dihydro-4-oxo-3-quinolinecarboxylic acid

In the same manner as descnbed in Example 2, except that 3-methylthiomethylpyrrolidine hydroiodide and 1-cyclopropyl-5-amino-6,7,8-trifluoro-1,4-dihydro-4-oxo-3-quinolinecarboxylic acid were used in place of 3-methylthiopyrrolidine hydroiodide and 1-cyclopropyl-6,7,8-trifluoro-1,4-dihydro-4-oxo-3-quinolinecarboxylic acid, the title compound was prepared as yellow solid.
- m.p. :: 158-163°C
- ¹H NMR(ppm, CDCl₃) :: 0.94 ∼ 1.20(m, 4H), 1.43 ∼ 2.53(m, 3H), 2.14(s, 3H), 2.59(s, 2H), 3.47 - 4.21(m, 5H), 6.36(br, 2H), 8.54(s, 1H), 14.83(br, 1H)

### Example 10

### Preparation of 1-cyclopropyl-6,8-difluoro-7-(3-mercaptomethylpyrrolidinyl)-1,4-dihydro-4-oxo-3-quinolinecarboxylic acid

In the same manner as described in Example 2, except that 3-mercaptomethylpyrrolidine trifluoroacetate was used in place of 3-methylthiopyrrolidine hydroiodide, the title compound was prepared as pale yellow solid.
- m.p. :: 238 ∼ 243°C (decomp.)
- ¹H NMR(ppm, NaOD/D₂O) :: 0.94 - 1.23(m, 4H), 1.63 - 3.01(m, 5H), 2.14(s, 3H), 3.23 - 4.01(m, 5H), 7.50(d, 1H), 8.37(s, 1H)

### Example 11

### Preparation of 1-cyclopropyl-6,8-difluoro-7-[3-(2-pyridylthiomethyl)-pyrrolidinyl]-1,4-dihydro-4-oxo-3-quinolinecarboxylic acid

In the same manner as described in Example 9, except that 3-(2-pyridylthiomethyl)-pyrrolidine trifluoroacetate was used in place of 3-methylthiomethylpyrrolidine hydroiodide, the title compound was prepared as pale yellow solid.
- m.p. :: 196 ∼ 200°C.
- ¹H NMR(ppm, CDCl₃) :: 0.91 ∼ 1.46(m, 4H), 1.50 ∼ 2.82(m, 3H), 3.39(d, 2H), 3.30 ∼ 4.24(m, 5H), 6.72 ∼ 7.94(m, 4H), 8.42(d, 1H), 8.67(s, 1H), 14.83(br, 1H)

### Example 12

### Preparation of 1-cyclopropyl-6,8-difluoro-7-(3-phenylthiomethylpyrrolidinyl)-1,4-dihydro-4-oxo-3-quinolinecarboxylic acid

In the same manner as described in Example 9, except that 3-phenylthiomethylpyrrolidine trifluoroacetate was used in place of 3-methylthiomethylpyrrolidine hydroiodide, the title compound was prepared as pale yellow solid.
- m.p. :: 160∼164°C
- ¹H NMR(ppm, CDCl₃) :: 0.90 ∼ 1.24(m, 4H), 1.32 ∼ 2.80(m, 3H), 3.10(d, 2H), 3.30 ∼ 4.32(m, 5H), 7.30(s, 5H), 7.80(dd, 1H), 8.69(s, 1H), 14.80(br, 1H)

### Example 13

### Preparation of 1-(2,4-difluorophenyl)-6,8-difluoro-7-[3-(2-pyridylthiomethyl) pyrrolidinyl]-1,4-dihydro-4-oxo-3-quinolinecarboxylic acid

In the same manner as described in Example 9, except that 3-(2-pyridylthiomethyl)pyrrolidine trifluoroacetate and 1-(2,4-difluorophenyl)-6,7,8-trifluoro-1,4-dihydro-4-oxo-3-quinolinecarboxylic acid were used in place of 3-methylthiomethylpyrrolidine hydroiodide and 1-cyclopropyl-6,7,8-trifluoro-1,4-dihydro-4-oxo-3-quinolinecarboxylic acid, the title compound was prepared as pale yellow solid.
- m.p. :: 202 ∼ 206°C
- ¹H NMR(ppm, CDCl₃) :: 1.84 - 2.72(m, 3H), 3.32(d, 2H), 3.24 ∼ 4.12(m, 5H), 6.81 ∼ 8.34(m, 8H), 8.39(s, 1H), 14.66(br, 1H)

### Example 14

### Preparation of 1-(2,4-difluorophenyl)-6-fluoro-7-(3-phenylthiomethylpyrrolidinyl) 1,4-dihydro-4-oxo-3-quinolinecarboxylic acid

In the same manner as described in Example 9, except that 3-phenylthiomethylpyrrolidine trifluoroacetate and 1-(2,4-difluorophenyl)-6,7-difluoro-1,4-dihydro-4-oxo-3-quinolinecarboxylic acid were used in place of 3-methylthiomethylpyrrolidine hydro iodide and 1-cyclopropyl-6,7,8-trifluoro-1,4-dihydro-4-oxo-3-quinolinecarboxylic acid, the title compound was prepared as pale yellow solid.
- m.p. :: 180 ∼184°C
- ¹H NMR(ppm, CDCl₃) :: 1.56 ∼ 2.82(m, 3H), 3.02(d, 2H), 3,10 ∼ 4.12(m, 4H), 5.76(d, 1H), 6.90 ∼ 8.20(m, 4H), 7.27(s, 5H), 8.42(s, 1H), 15.05(br, 1H)

The typical examples of the present compounds were tested for antimicrobial activity in vitro against twenty strains of Gram-positive and Gram-negative microorganisms.

The antibacterial activity in vitro is shown in Table 1. The numerals in the table 1 show minimum inhibitory concentrations(MIC in *µg/ml*). The minimum inhibitory concentrations were determined according to the agar dilution method recommended by Japan Society of Chemotherapy[Chemotherapy., 29, 76 (1981)].

**Table 1.**

| In vitro Antibacterial Activity, MIC (*µg/ml*) | | | | | | | |
|---|---|---|---|---|---|---|---|
| No. | example compound | | 1 | 2 | 3 | 9 | CIP |
| 1 | *S. pyogenes* | A 308 | 1.563 | 0.195 | 0.781 | 0.195 | 1.563 |
| 2 | *S. pyogenes* | A 77 | 0.781 | 0.098 | 1.563 | 0.391 | 0.195 |
| 3 | *S. faecium* | MD 86 | 0.781 | 0.195 | 0.781 | 1.195 | 0.781 |
| 4 | *S. aureus* | SG 511 | 0.025 | 0.002 | 0.008 | 0.004 | 0.195 |
| 5 | *S. aureus* | 285 | 0.025 | 0.002 | 0.013 | 0.025 | 0.195 |
| 6 | *S. aureus* | 503 | 0.025 | 0.002 | 0.007 | 0.007 | 0.391 |
| 7 | *E. coli* | O 55 | 0.025 | 0.013 | 0.195 | 0.013 | 0.013 |
| 8 | *E. coli* | DC 0 | 3.125 | 0.781 | 6.25 | 3.125 | 0.195 |
| 9 | *E. coli* | DC 2 | 0.781 | 0.781 | 3.125 | 0.195 | 0.098 |
| 10 | *E. coli* | TEM | 0.391 | 0.391 | 3.125 | 0.025 | 0.098 |
| 11 | *E. coli* | 1507E | 1.563 | 0.391 | 3.125 | 0.025 | 0.049 |
| 12 | *P. aeruginosa* | 9027 | 1.563 | 0.781 | 3.125 | 3.125 | 0.098 |
| 13 | *P. aeruginosa* | 1592E | 1.563 | 0.781 | 3.125 | 3.125 | 0.195 |
| 14 | *P. aeruginosa* | 1771 | 0.781 | 0.391 | 1.563 | 1.563 | 0.195 |
| 15 | *P. aeruginosa* | 1771M | 0.391 | 0.098 | 1.563 | 0.781 | 0.049 |
| 16 | *S. typhimurium* | | 0.195 | 0.049 | 0.391 | 0.049 | 0.013 |
| 17 | *K. oxytoca* | 1082E | 0.098 | 0.007 | 0.098 | 1.563 | 0.002 |
| 18 | *K. aerogenes* | 1522E | 0.391 | 0.098 | 0.781 | 3.125 | 0.013 |
| 19 | *E. cloacae* | P99 | 0.195 | 0.098 | 0.391 | 0.049 | 0.013 |
| 20 | *E. cloacae* | 1321E | 0.195 | 0.025 | 0.391 | 0.049 | 0.007 |
| CIP = Ciprofloxacin | | | | | | | |

The compound of example 2 was orally administered to determine the LD₅₀ for a group of male mice. The result was proved to be LD₅₀ > 3000 mg/kg. The antibacterial activity for MRSA(Methicillin Resistant Staphyloccocus Aureus) was tested by the compound of example 2. The results are shown in Table 2.

**Table 2.**

| Antibacterial Activity for MRSA (*µg/ml*) | | | |
|---|---|---|---|
| No. | Fungus | example 2 | CIP. |
| 1 | *Staphylococcus aureus* 88 E | 0.004 | 0.391 |
| 2 | *Staphylococcus aureus* 121E | 0.013 | 0.391 |
| 3 | *Staphylococcus aureus* 208E | 0.025 | 0.195 |
| 4 | *Staphylococcus aureus* 256E | 0.013 | 0.391 |
| 5 | *Staphylococcus aureus* 690E | 0.004 | 0.195 |
| 6 | *Saphylococcus aureus* 692E | 0.004 | 0.195 |
| 7 | *Staphylococcus aureus* 693E | 0.007 | 0.391 |
| 8 | *Staphylococcus aureus* 694E | 0.013 | 0.391 |
| 9 | *Staphylococcus aureus* 695E | 0.013 | 0.391 |
| 10 | *Staphylococcus aureus* 697E | 0.013 | 0.195 |
| 11 | *Staphylococcus aureus* 701E | 0.013 | 0.391 |
| 12 | *Staphylococcus aureus* 703E | 0.007 | 0.195 |
| 13 | *Staphylococcus aureus* 705E | 0.013 | 0.391 |
| 14 | *Staphylococcus aureus* 706E | 0.013 | 0.391 |
| 15 | *Staphylococcus aureus* 707E | 0.007 | 0.391 |
| 16 | *Staphylococcus aureus* 708E | 0.007 | 0.098 |
| 17 | *Staphylococcus aureus* 711E | 0.013 | 0.098 |
| 18 | *Staphylococcus aureus* 714E | 0.007 | 0.195 |
| 19 | *Staphylococcus aureus* 752E | 0.013 | 0.195 |
| CIP = Ciprofloxacin | | | |

## Claims

1. A compound of following structural formula (I) or pharmaceutically acceptable salts thereof. 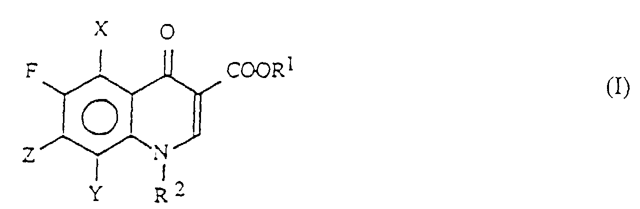 wherein
R¹ is hydrogen or alkyl group having one to six carbon atoms,
R² is alkyl group having one to four cabon atoms, haloalkyl group having one to four carbon atoms, cycloalkyl group having three to six carbon atoms, alkenyl group having two to four carbon atoms, or phenyl group which is optionally mono or disubstituted by fluorine atoms,
X is hydrogen, amino, or halogen,
Y is hydrogen, or halogen,
Z is 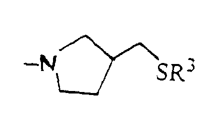 (wherein R³is hydrogen, alkyl group having one to four carbon atoms, haloalkyl group having one to three carbon atoms, cycloalkyl group having three to six carbon atoms, phenyl, or 2-pyridyl group).

2. A compound according to claim 1, wherein R¹ is hydrogen.

3. A compound according to claim 1, wehrein X is hydrogen, or amino.

4. A compound according to claim 1, wherein Y is hydrogen, fluorine, or chlorine.

5. A compound according to claim 1, wherein R² is cyclopropyl, or 2,4-difluorophenyl.

6. A compound according to claim 1, wherein R³ is methyl, ethyl, phenyl, or 2-pyridyl.

7. A compound according to claim 1 an selected from the groups consisting of:
1-cyclopropyl-6-fluoro-7-(3-methylthiomethylpyrrolidinyl)-1,4-dihydro-4-oxo-3-quinolinecarboxylic acid,
1-cyclopropyl-6,8-difluoro-7-(3-methylthiomethylpyrrolidinyl)-1,4-dihydro-4-oxo-3-quinolinecarboxylic acid,
1-cyclopropyl-5,6,8-trifluoro-7-(3-methylthiomethylpyrrolidinyl)-1,4 dihydro-4-oxo-3-quinolinecarboxylic acid,
1-cyclopropyl-6,8-difluoro-7-(3-ethylthiomethylpyrrolidinyl)-1,4-dihydro-4-oxo-3-quinolinecarboxylic acid,
1-cyclopropyl-5,6,8-trifluoro-7-(3-ethylthiomethylpyrrolidinyl)-1,4-dihydro-4-oxo-3-quinolinecarboxylic acid,
1-cyclopropyl-6-fluoro-8-chloro-7-(3-methylthiomethylpyrrolidinyl)-1,4-dihydro-4-oxo-3-quinolinecarboxylic acid.
1-(2,4-difluorophenyl)-6,8-difluoro-7-(3-methylthiomethylpyrrolidinyl)-1,4-dihydro-4-oxo-3-quinolinecarboxylic acid.
1-(2,4-difluorophenyl)-5,6,8-trifluoro-7-(3-ethylthiomethylpyrrolodinyl)-1,4-dihydro-4-oxo-3-quinolinecarboxylic acid,
1-cyclopropyl-5-amino-6,8-difluoro-7-(3-methylthiomethylpyrrolidinyl)-1,4-dihydro-4-oxo-3-quinolinecarboxylic acid,
1-cyclopropyl-6,8-difluoro-7-(3-mercaptomethylpyrrolidinyl)-1,4-dihydro-4-oxo-3-quinolinecarboxylic acid,
1-cyclopropyl-6,8-difluoro-7-[3-(2-pyridylthiomethyl)pyrrolidinyl]-1,4-dihydro-4-oxo-3-quinolinecarboxylic acid,
1-(2,4-difluorophenyl)-6,8-difluoro-7-[3-(2-pyridyithiomethyl)pyrrolidinyl]-1,4-dihydro-4-oxo-3-quinolinecarboxylic acid,
1-cyclopropyl-6,8-difluoro-7-(3-phenylthiomethylpyrrolidinyl)-1,4-dihydro-4-oxo-3-quinolinecarboxylic acid,
1-(2,4-difluorophenyl)-6-fluoro-7-(3-phenylthiomethylpyrrolidinyl)-1,4-dihydro-4-oxo-3-quinolinecarboxylic acid, and pharmaceutically acceptable salts thereof.

8. A process for the preparation of compounds of structural formula of claim 1 by reacting a compound of structural formula(II) with a compound of structural formula(III). 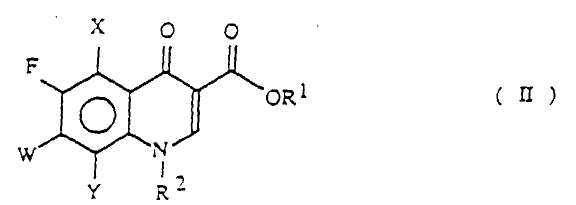 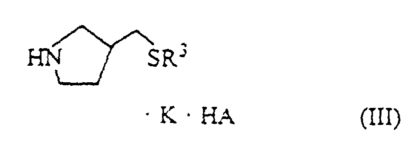 wherein
R¹, R², R³, X, Y and Z are defined as in claim 1 and
W is halogen,
HA is organic acid or inorganic acid,
K is 0 or 1.

9. A pharmaceutical composition comprising an antibacterially effective amount of compound according to claim 1 together with a pharmaceutically acceptable carrier.

## Patentansprüche

1. Verbindung der folgenden Strukturformel (I) oder pharmazeutisch verträgliche Salze hiervon 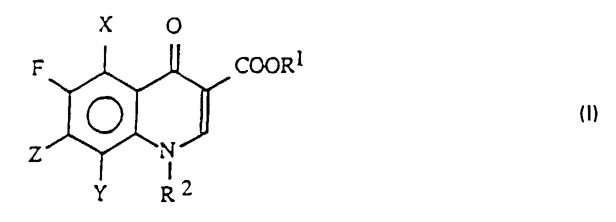 worin
R¹ Wasserstoff oder eine Alkylgruppe mit 1 bis 6 Kohlenstoffatomen ist,
R² eine Alkylgruppe mit 1 bis 4 Kohlenstoffatomen, eine Halogenalkylgruppe mit 1 bis 4 Kohlenstoffatomen, eine Cycloalkylgruppe mit 3 bis 6 Kohlenstoffatomen, eine Alkenylgruppe mit 2 bis 4 Kohlenstoffatomen oder eine Phenylgruppe, die gegebenenfalls durch Fluoratome mono- oder disubstituiert ist, ist,
X Wasserstoff, Amino oder Halogen ist,
Y Wasserstoff oder Halogen ist und
Z 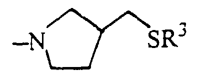
(worin
R³ Wasserstoff, eine Alkylgruppe mit 1 bis 4 Kohlenstoffatomen, eine Halogenalkylgruppe mit 1 bis 3 Kohlenstoffatomen, eine Cycloalkylgruppe mit 3 bis 6 Kohlenstoffatomen, Phenyl oder eine 2-Pyridylgruppe ist) ist.

2. Verbindung nach Anspruch 1, worin R¹ Wasserstoff ist.

3. Verbindung nach Anspruch 1, worin X Wasserstoff oder Amino ist.

4. Verbindung nach Anspruch 1, worin Y Wasserstoff, Fluor oder Chlor ist.

5. Verbindung nach Anspruch 1, worin R² Cyclopropyl oder 2,4-Difluorphenyl ist.

6. Verbindung nach Anspruch 1, worin R³ Methyl, Ethyl, Phenyl oder 2-Pyridyl ist.

7. Verbindung nach Anspruch 1 und ausgewählt aus den Gruppen bestehend aus:
1-Cyclopropyl-6-fluor-7-(3-methylthiomethylpyrrolidinyl)-1,4-dihydro-4-oxo-3-chinolin-carbonsäure,
1-Cyclopropyl-6, 8-difluor-7-(3-methylthiomethylpyrrolidinyl)-1 ,4-dihydro-4-oxo-3-chinolincarbonsäure,
1-Cyclopropyl-5,6,8-trifluor-7-(3-methylthiomethylpyrrolidinyl)-1,4-dihydro-4-oxo-3-chinolincarbonsäure,
1-Cyclopropyl-6,8-difluor-7-(3-ethylthiomethylpyrrolidinyl)-1,4-dihydro-4-oxo-3-chinolincarbonsäure,
1-Cyclopropyl-5,6,8-trifluor-7-(3-ethylthiomethylpyrrolidinyl)-1,4-dihydro-4-oxo-3-chinolincarbonsäure,
1-Cyclopropyl-6-fluor-8-chlor-7-(3-methylthiomethylpyrrolidinyl)-1,4-dihydro-4-oxo-3-chinolincarbonsäure,
1-(2,4-Difluorphenyl)-6,8-difluor-7-(3-methylthiomethylpyrrolidinyl)-1,4-dihydro-4-oxo-3-chinolincarbonsäure,
1-(2,4-Difluorphenyl)-5,6,8-trifluor-7-(3-ethylthiomethylpyrrolidinyl)-1,4-dihydro-4-oxo-3-chinolincarbonsäure,
1-cyclopropyl-5-amino-6,8-difluor-7-(3-methylthiomethylpyrolidinyl)-1,4-dihydro-4-oxo-3-chinolincarbonsäure,
1-Cyclopropyl-6,8-difluor-7-(3-mercaptomethylpyrrolidinyl)-1,4-dihydro-4-oxo-3-chinolincarbonsäure,
1-Cyclopropyl-6,8-difluor-7-[3-(2-pyridylthiomethyl)-pyrrolidinyl)-1,4-dihydro-4-oxo-3-chinolincarbonsäure,
1-(2,4-Difluorphenyl)-6,8-difluor-7-[3-(2-pyridylthiomethyl)-pyrrolidinyl]-1,4-dihydro-4-oxo-3-chinolincarbonsäure,
1-Cyclopropyl-6,8-difluor-7-(3-phenylthiomethylpyrrolidinyl)-1,4-dihydro-4-oxo-3-chinolincarbonsäure,
1-(2,4-Difluorphenyl)-6-fluor-7-(3-phenylthiomethylpyrrolidinyl)-1,4-dihyro-4-oxo-3-chinolincarbonsäure
und pharmazeutisch verträgliche Salze hiervon.

8. Verfahren zur Herstellung von Verbindungen der Strukturformel von Anspruch 1 durch Umsetzung einer Verbindung der Strukturformel (II) mit einer Verbindung der Strukturformel (III) 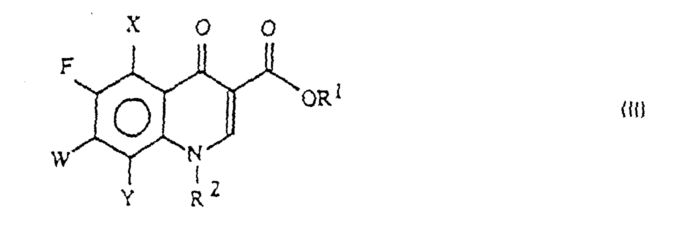 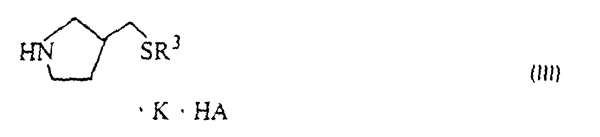 worin
R¹, R², R³, X, Y und Z wie in Anspruch 1 definiert sind,
W Halogen ist,
HA eine organische Säure oder anorganische Säure ist und
K 0 oder 1 ist.

9. Pharmazeutische Zusammensetzung mit einer antibakteriell wirksamen Menge von Verbindung nach Anspruch 1 zusammen mit einem pharmazeutisch verträglichen Träger.

## Revendications

1. Composé de formule générale suivante (I) ou ses sels acceptables d'un point de vue pharmaceutique. 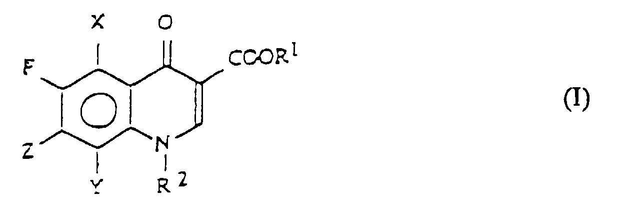 dans laquelle
R¹ est l'hydrogène ou un groupe alkyle ayant de un à six atomes de carbone,
R² est un groupe alkyle ayant de un à quatre atomes de carbone, un groupe haloalkyle ayant de un à quatre atomes de carbone, un groupe cycloalkyle ayant de trois à six atomes de carbone, un groupe alcényle ayant de deux à quatre atomes de carbone, ou un groupe phényle qui est éventuellement mono- ou disubstitué par des atomes de fluor.
X est l'hydrogène, un groupe amino ou un halogène,
Y est l'hydrogène ou un halogène;
Z est 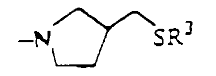 (dans laquelle R³ est l'hydrogène ou un groupe alkyle ayant de un à quatre atomes de carbone, un groupe haloalkyle ayant de un à trois atomes de carbone, un groupe cycloalkyle ayant de trois à six atomes de carbone, un groupe phényle ou un groupe 2-pyridyle).

2. Composé selon la revendication 1, dans lequel R¹ est l'hydrogène.

3. Composé selon la revendication 1, dans lequel X est l'hydrogène ou un groupe amino.

4. Composé selon la revendication 1, dans lequel Y est l'hydrogène, le fluor ou le chlore.

5. Composé selon la revendication 1, dans lequel R¹ est un groupe cyclopropyle ou 2,4-difluorophényle.

6. Composé selon la revendication 1, dans lequel R³ est un groupe méthyle, éthyle, phényle ou 2-pyridyle,

7. Composé selon la revendication 1, choisi dans le groupe constitué par :
l'acide 1-cyclopropyl-6-fluoro-7-(3-méthylthiométhylpyrrolidinyl)-1,4-dihydro-4-oxo-3-quinoléinecarboxylique,
l'acide 1-cyclopropyl-6,8-difluoro-7-(3-méthylthiométhylpyrrolidinyl)-1,4-dihydro-4-oxo-3-quinoléinecarboxylique,
l'acide 1-cyclopropyl-5,6,8-trifluoro-7-(3 méthylthiométhylpyrrolidinyl)-1,4-dihydro-4-oxo-3-quinoléinecarboxylique,
l'acide 1-cyclopropyl-6,8-difluoro-7-(3-éthylthiométhylpyrrolidinyl)-1,4-dihydro-4-oxo-3-quinoléinecarboxylique,
l'acide 1-cyclopropyl-5,6,8-trifluoro-7-(3-éthylthiométhylpyrrolidinyl)-1,4-dihydro-4-oxo-3-quinoléinecarboxylique,
l'acide 1-cyclopropyl-6-fluoro-8-chloro-7-(3-méthylthiométhylpyrrolidinyl)-1,4-dihydro-4-oxo-3-quinoléinecarboxylique,
l'acide 1-(2,4-difluorophényl)-6,8-difluoro-7-(3-méthylthiométhylpyrrolidinyl)-1,4-dihydro-4-oxo-3-quinoléinecarboxylique,
l'acide 1-(2,4-difluorophenyl)-5,6,8-trifluoro-7-(3-éthylthiométhylpyrrolidinyl)-1,4-dihydro-4-oxo-3-quinoléinecarboxylique,
l'acide 1-cyclopropyl-5-amino-6,8-difluoro-7-(3-méthylthiométhylpyrrolidinyl)-1,4-dihydro-4-oxo-3-quinoléinecarboxylique,
l'acide 1-cyclopropyl-6,8-difluoro-7-(3-mercaptométhylpyrrolidinyl)-1,4-dihydro-4-oxo-3-quinoléinecarboxylique,
l'acide 1-cyclopropyl-6,8-difluoro-7-[3(2-pyridylthiométhyl)pyrrolidinyl]-1,4-dihydro-4-oxo-3-quinoléinecarboxylique,
l'acide 1-(2,4-difluorophényl)-6,8-difluoro-7-[3(2-pyridylthiométhyl)pyrrolidinyl]-1,4-dihydro-4-oxo-3-quinoléinecarboxylique,
l'acide 1-cyclopropyl-6,8-difluoro-7-(3-phénylthiométhylpyrrolidinyl)-1,4-dihydro-4-oxo-3-quinoléinecarboxylique,
l'acide 1-(2,4-difluorophényl)-6-fluoro-7-(3-phénylthiométhyl)pyrrolidinyl)-1,4-dihydro-4-oxo-3-quinoléinecarboxylique,
et leurs sels acceptables d'un point de vue pharmaceutique.

8. Procédé de préparation de composés de formule générale selon la revendication 1 comprenant l'étape consistant à faire réagir un composé de formule générale (II) avec un composé de formule générale (III). 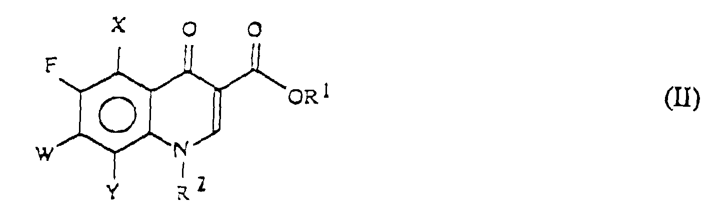 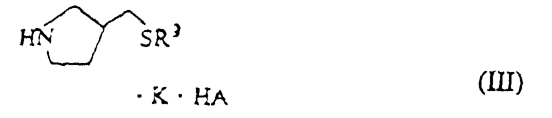 dans laquelle
R¹ R², R³, X, Y et Z sont tels que définis à la revendication 1 et
W est un halogène,
HA est un acide organique ou minéral,
K vaut 0 ou 1.

9. Composition pharmaceutique comprenant une quantité efficace d'un point de vue antibactérien d'un composé selon la revendication 1 associé à un support acceptable d'un point de vue pharmaceutique.
